# EUROPEAN PATENT APPLICATION

(11) **EP 4 563 072 A1**
(43) Date of publication of application: **04.06.2025**
(21) Application number: 23846252.7
(22) Date of filing: 13.07.2023
(51) Int. Cl.: A61B 3/16

(54) **OPHTHALMOLOGICAL DEVICE**

(30) Priority: 25.07.2022 JP 2022117898
(71) Applicant: TOPCON CORPORATION, Tokyo 174-8580 (JP)
(72) Inventor: MARUYAMA, Hirotake, Tokyo 174-8580 (JP); OMIYA, Takeshi, Tokyo 174-8580 (JP)
(74) Representative: Schmitt-Nilson Schraud Waibel Wohlfrom Patentanwälte Partnerschaft mbB
(86) International application number: PCT/JP2023/025840
(87) International publication number: WO 2024/024521

(57) **Abstract**

In an ophthalmological device measuring ocular pressure using high pressure gas, action noise and shock are reduced. The ophthalmological device includes: a high pressure bomb 101 which generates high pressure gas continuously; a pressure storing tank 104 which stores the high pressure gas supplied from the high pressure bomb 101; a pressure regulator 103 which is arranged between the high pressure bomb 101 and the pressure storing tank 104 and which supplies high pressure air supplied from the high pressure bomb 101 to the pressure storing tank 104 in a condition maintained at a certain pressure; a nozzle 107 which is connected to the pressure storing tank 104 and which blows high pressure air onto the eye to be tested 200; and an electromagnetic valve 105 which is arranged between the pressure storing tank 104 and the nozzle 107.

## Description

### Technical Field

The present invention relates to an ophthalmological device to measure ocular pressure.

### Background Art

An ophthalmological device of the non-contact type is known in which air is blown onto an eyeball and deformation of the eyeball during the blowing of air is optically measured so as to measure ocular pressure (For example, see Patent Document 1).

Patent Document 1: Japanese Unexamined Patent Application Publication No. 2008-237516

### Summary of Invention

In a conventional ophthalmological device for measuring ocular pressure, high pressure gas is produced using a cylinder and air is blown onto an eyeball to be measured. This structure has problems in that the noise of the action and the shock during the action may disturb the test subject.

In view of such circumstances, an object of the present invention is to reduce the action noise and the shock in an ophthalmological device in which ocular pressure is measured using high pressure gas.

An aspect of the present invention is an ophthalmological device including a high pressure gas supplying means for generating high pressure gas continuously, a pressure storing tank for storing the high pressure gas supplied from the high pressure gas supplying means, a pressure stabilizing means for maintaining an inner pressure of the pressure storing tank at a predetermined value, a nozzle connected to the pressure storing tank and blowing of the high pressure gas onto an eye of a subject, and a primary ON-OFF valve arranged between the pressure storing tank and the nozzle.

In the present invention, an aspect can be mentioned in which the high pressure gas supplying means is a high pressure bomb storing high pressure gas, and an aspect can be mentioned in which the high pressure gas supplying means is a pump. In the present invention, an aspect can be mentioned in which the pump generates high pressure air flow, including pulsing flow, using a diaphragm.

In the present invention, an aspect can be mentioned in which high pressure gas is started to be blown onto the eye of a subject by making the primary ON-OFF valve open, and then high pressure gas is stopped being blown onto the eye of a subject by making the primary ON-OFF valve close.

In the present invention, an aspect can be mentioned in which a secondary ON-OFF valve is arranged between the high pressure gas supplying means and the pressure storing tank; and high pressure gas is blown from the nozzle onto the eye of a subject by making an inside of the pressure storing tank be at a certain high pressure state under conditions in which the primary ON-OFF valve is closed and the secondary ON-OFF valve is opened, then, by making the secondary ON-OFF valve closed, and after that, by making the primary ON-OFF valve opened.

### Effects of Invention

According to the present invention, the action noise and the shock can be reduced in the ophthalmological device for measuring ocular pressure using high pressure gas.

### Brief Description of Drawings

Fig. 1 is a conceptual diagram showing the ophthalmologic device of an embodiment.
Fig. 2 is a conceptual diagram showing the ophthalmologic device of another embodiment.
Fig. 3 is a conceptual diagram showing the ophthalmologic device of another embodiment.

### Embodiment of Invention

### 1. First Embodiment

### (Structure)

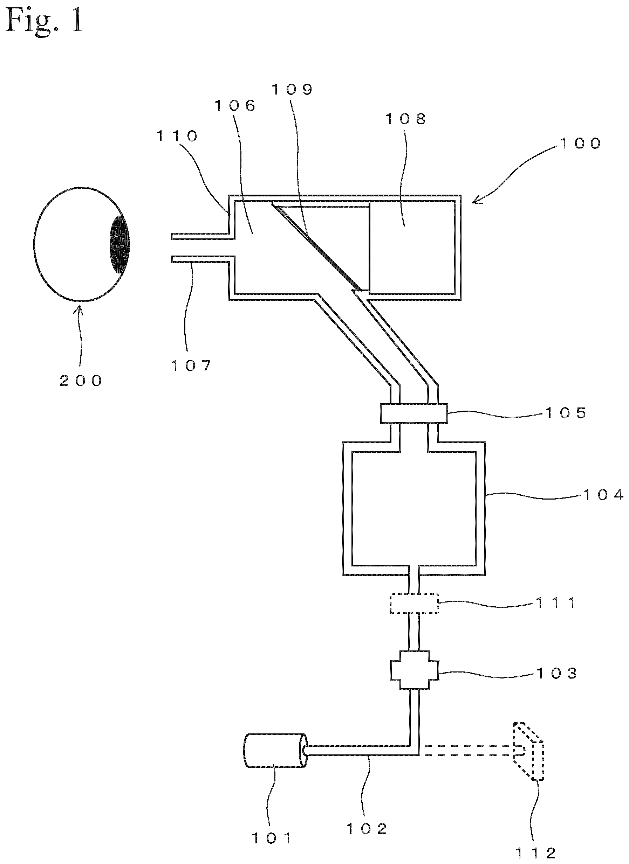

Fig. 1 shows an ophthalmological device 100 in which the present invention is employed. The ophthalmological device 100 blows high pressure air onto an eye to be tested 200, optically detects the degree of deformation of the eye to be tested 200 which is deformed by air pressure, and measures ocular pressure of the eye to be tested 200. This mechanism is similar to that in a conventional ophthalmological device.

The ophthalmological device 100 includes a high pressure bomb 101 which is a high pressure gas supplying means to continuously generate high pressure gas. Here, the pressure in the high pressure means is higher than atmospheric pressure. The high pressure bomb 101 stores compressed air.

As the high pressure bomb 101, one is selected in which an inner pressure thereof is higher than the maximal value of the inner pressure of a pressure storing tank 104 mentioned below. In this embodiment, the kind of gas stored in the high pressure bomb 101 is air; however, carbon dioxide gas, nitrogen gas or the like can be used.

In one example, the amount blown once onto the eye to be tested in a recent ocular pressure measuring device is about 3.3 mL. Assuming this amount is used, in a case in which a commercially available mini CO₂ cartridge (diameter: 40 mm, length: 134 mm, volume: 98 ml, inner pressure: 41.7 MPa) is used, it can be calculated that blowing is possible about 12,000 times.

A pressure regulator 103 which is a pressure stabilizing means is connected to the high pressure bomb 101 via a pipe 102. High pressure air supplied from the high pressure bomb 101 is supplied to the pressure storing tank (accumulator) 104 via the pressure regulator 103.

In the pressure regulator 103, side of the high pressure bomb 101 corresponds to input side and the side of the pressure storing tank 104 corresponds to the output side. The pressure regulator 103 has a function maintaining pressure of the output side (inner pressure of the pressure storing tank 104) at a constant level, under conditions of "input side pressure > output side pressure". A commercially available one can be used as the pressure regulator 103.

The pressure storing tank 104 stores air in which the pressure is controlled to be higher than atmosphere pressure by the pressure regulator 103.

The amount contained in the pressure storing tank 104 is set to have a sufficient volume with respect to amount (volume) of air blown from the nozzle 107 onto the eye to be tested 200. In this case, by controlling timing of closing an electromagnetic valve 105, the amount of air blown from the nozzle 107 onto the eye to be tested 200 is controlled.

The pressure storing tank 104 is airtight, and it is connected to an air chamber 106 via the electromagnetic valve 105. The nozzle 107 which is for blowing high pressure air onto the eye to be tested 200 is connected to the air chamber 106.

The ophthalmological device 100 includes measuring optical system 108. The measuring optical system 108 includes a light emitting part of measuring light and a light receiving part of reflected light from the eye to be tested 200. Measuring light which is emitted at the light emitting part in the measuring optical system 108 is irradiated onto the eye to be tested 200 via light transmitting parts 109 and 110, and the reflection light thereof is received at the light receiving part in the measuring optical system 108.

### (Principal of measurement of ocular pressure)

High pressure air is blown from the nozzle 107 onto the eye to be tested 200 in a condition in which the measuring light is irradiated onto the eye to be tested 200. During this, the eyeball to be tested 200 is deformed by the pressure of high pressure air. Practically, a surface of the eye to be tested is deformed in the following order: convex shape, flat, and concave shape. Accompanied by this deformation, the amount of reflected light from the eye to be tested 200 may vary.

Using a standard eye model, relationships between change in the amount of reflected light and ocular pressure is preliminarily obtained. Actual change in amount of reflected light from the eye to be tested 200 is applied to this relationship, pressure on the eye to be tested 200 can be calculated. This is a basic principle in measuring ocular pressure in the ophthalmological device 100. This is as the same as in conventional measurement of ocular pressure.

### (Steps of measurement)

First, high pressure air is supplied from the high pressure bomb 101 to the pressure storing tank 104 in a condition in which the electromagnetic valve 105 is closed, so that pressure inside the pressure storing tank 104 is set at a predetermined pressure. This pressure can be determined by controlling the pressure regulator 103. The inner pressure of the pressure storing tank 104 is determined considering pressure and amount of high pressure air blown from the nozzle 107 onto the eye to be tested.

When the inner pressure of the pressure storing tank 104 reaches the predetermined pressure, high pressure air can be blown from the nozzle 107 onto the eye to be tested 200. In this situation, if the electromagnetic valve 105 is opened, high pressure air in the pressure storing tank 104 moves to the air chamber 106, and the high pressure air is blown from the nozzle 107 at the eye to be tested 200.

Here, by closing the electromagnetic valve 105 at an appropriate timing, the amount of air which is blown from the nozzle 107 is controlled. As a method to determine the timing to close the electromagnetic valve 105, a method in which the valve is closed in a step after a predetermined time has passed since opening, a method in which the valve is closed based on change in intensity of reflected light from the eye to be tested 200 or the like can be mentioned. As a practical example of the latter method, for example, a method can be mentioned in which a step at which a surface of the eye to be tested 200 has flattened (amount of reflection light is maximal) is optically detected, and the valve is closed based on this.

The duration of blowing of high pressure air from the nozzle 107 is set to be about 400 µs to 2000 µs (this duration is due to a relationship with pressure). As the electromagnetic valve 105, one is selected which can realize this opening-closing action.

### (Superiority)

The ophthalmological device 100 includes the high pressure bomb 101 generating high pressure gas continuously, the pressure storing tank 104 storing the high pressure gas supplied from the high pressure bomb 101, the pressure regulator 103 which is arranged between the high pressure bomb 101 and the pressure storing tank 104, which is a pressure stabilizing means maintaining inner pressure of the pressure storing tank 104 at a certain value, and which supplies high pressure air supplied from the high pressure bomb 101 to the pressure storing tank 104 in a condition that stabilizes at a certain pressure, the nozzle 107 which is connected to the pressure storing tank 104 and which blows high pressure gas onto the eye to be tested 200, and the electromagnetic valve 105 is arranged between the pressure storing tank 104 and the nozzle 107.

In this structure, since only the electromagnetic valve acts during blowing of high pressure air onto the eye to be tested 200, reduction in noise and shock can be realized. Furthermore, since vibration can be reduced during the action, error in alignment in which axis of the nozzle 107 is misaligned with respect to the eye to be tested 200 by vibration can be reduced. Furthermore, a person to be tested may unfortunately have a physiological reaction (being surprised and then moving away) and discomfort if there is action noise or shock generated; however, these problems are avoided since action noise and shock can be reduced.

Furthermore, since the present invention has no return mechanism, which exists in a case in which a piston is used, there is no problem of tear droplets being draw in by the nozzle. Because of this feature, effects can be obtained in which infective disease and contamination in the nozzle and the air chamber are reduced. Furthermore, there is no mechanical action mechanism except for the electromagnetic valve, and high reliability can be obtained. Furthermore, the overall size can be minimized since the number of parts is small. Therefore, a portable ophthalmological device can be realized.

The inner pressure of the high pressure bomb 101 is progressively decreased as the number of uses increases, and output characteristics may vary. In this example, by maintaining constant pressure which is imparted to the pressure storing tank 104 by the pressure regulator 103, decrease and variation of output pressure of the high pressure bomb 101 due to decrease of inner pressure may be avoided from affecting the behavior of high pressure air which is blown from the nozzle 107.

### 2. Second Embodiment

In this example, the amount contained in the pressure storing tank 104 is set corresponding to the amount (volume) of air which is blown from the nozzle 107 to the eye to be tested 200. In addition, an electromagnetic valve 111 is added between the pressure storing tank 104 and the pressure regulator 103. One example of steps of action in this example case is explained as follows. First, high pressure air is sent from the high pressure bomb 101 via the pressure regulator 103 to the pressure storing tank 104 in a condition in which the electromagnetic valve 105 is closed and the electromagnetic valve 111 is opened, so that the inner pressure in the pressure storing tank 104 is set at a predetermined pressure.

After inner pressure in the pressure storing tank 104 reaches the predetermined pressure, the electromagnetic valve 111 is closed. Then, by opening the electromagnetic valve 105, high pressure air which is stored in the pressure storing tank 104 moves to the air chamber 106 and high pressure air is blown from the nozzle 107.

During this, setting values of volume and inner pressure of the pressure storing tank 104 are preliminarily determined so that amount and pressure of air which is blown from the nozzle 107 to the outside are predetermined values, respectively.

### 3. Third Embodiment

A structure is also possible in which a micro pump (micro blower) 112 is used as a source to supply high pressure air. In the micro pump 112, a diaphragm is driven by a piezoelectric element and air is ejected by this action of the diaphragm. Another structure can be employed as a pump. A structure is also possible in which a pump and a high pressure bomb are used in combination.

There are two kinds of micro pumps: one including a check valve and one not including a check valve. In a case in which the micro pump 112 of a type not including a check valve is used, the electromagnetic valve 111 is necessary. In this case, an electromagnetic valve or a check valve (not shown) can be arranged between the micro pump 112 and the pressure regulator 103, instead of the electromagnetic valve 111. In a case in which the micro pump 112 includes a check valve, a structure is also possible in which an electromagnetic valve or a check valve is not arranged between the micro pump 112 and the pressure storing tank 104.

The micro pump 112 has to increase pressure in the pressure storing tank 104 over a period of time, and it is not necessary to pump in a short period of time. Since pumping in short time is not necessary, the micro pump 112 can be quieter and can produce less shock. Furthermore, one of a small type can be employed as the micro pump 112. Furthermore, since high pressure air once stored in the pressure storing tank 104 is blown from the nozzle 107 to the eye to be tested 200, even if pulsing flow is generated in the micro pump 112, the effect does not reach the eye to be tested 200.

### 4. Fourth Embodiment

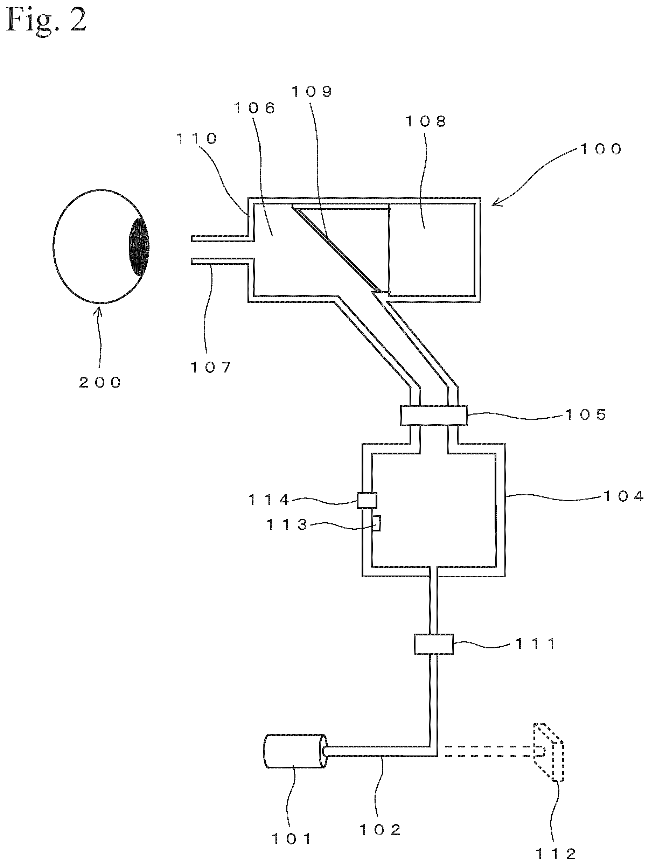

Fig. 2 shows a case in which the pressure storing tank 104 includes a function in which the inner pressure thereof is maintained constant. In this case, the pressure regulator 103 is not necessary (of course, combination use is possible). In this case, the pressure storing tank 104 includes a pressure sensor 113 which detects the inner pressure and an electromagnetic valve 114 which opens and closes based on the inner pressure of the pressure storing tank 104 detected by the pressure sensor 113.

Under conditions in which high pressure gas is supplied from the high pressure gas supplying means (high pressure bomb 101 or the micro pump 112) to the pressure storing tank 104, if the inner pressure of the pressure storing tank 104 is close to exceeding the predetermined set value, this is detected by the pressure sensor 113, and the electromagnetic valve 114 is opened. By this mechanism, the inner pressure of the pressure storing tank 104 is released, and the inner pressure is adjusted so as not to exceed the predetermined set value.

In addition, under conditions in which high pressure gas is supplied from the high pressure gas supplying means to the pressure storing tank 104, and the electromagnetic valve 114 is opened, if the inner pressure of the pressure storing tank 104 is nearly below the set value, this is detected by the pressure sensor 113, the electromagnetic valve 114 is closed, and the inner pressure of the pressure storing tank 104 is adjusted so as not to fall below the predetermined set value. Due to these dynamic adjustments being performed, the inner pressure of the pressure storing tank 104 is maintained constant.

In this structure, in a case in which the micro pump 112 does not including a check valve is used as the high pressure gas supplying means, it is necessary to arrange the electromagnetic valve 111 (or check valve) between the micro pump 112 and the pressure storing tank 104. In a case in which the micro pump includes the check valve, the electromagnetic valve 11 is not necessary. In a case in which the high pressure bomb 101 is used as the high pressure gas supplying means, the electromagnetic valve 111 is necessary.

### 5. Fifth Embodiment

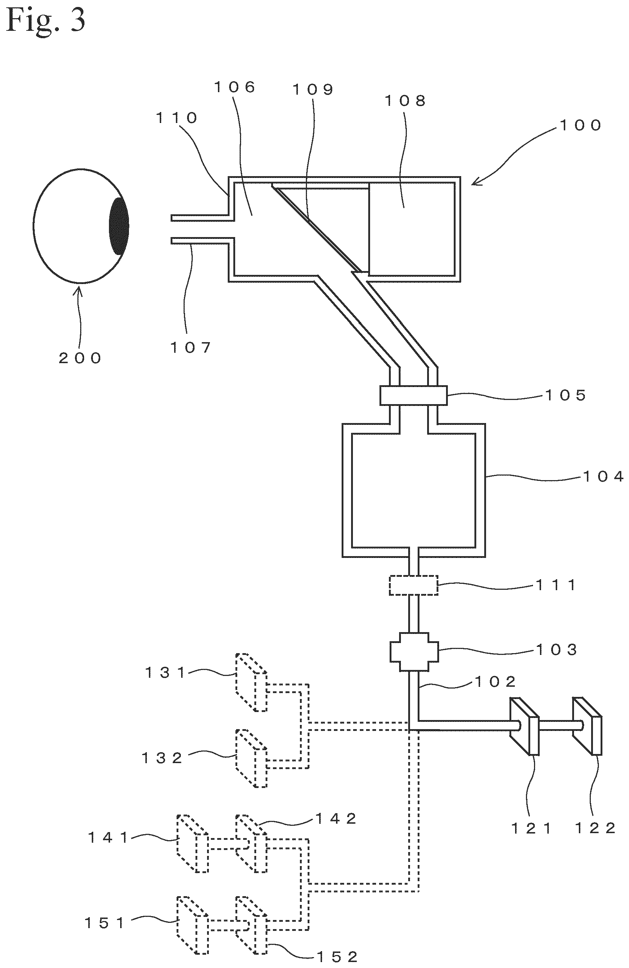

A structure in which multiple micro pumps are arranged is also possible. Fig. 3 shows an example in which multiple micro pumps are arranged in series. It should be noted that portions other than the micro pumps are as same as in Fig. 1. The present embodiment can be applied to the structure of Fig. 2.

Fig. 3 shows the example in which two micro pumps 121 and 122 are connected in series. By connecting multiple micro pumps in series, pressure of expelled gas can be increased. The number of micro pumps connected in series can be two or more, and the number can be determined depending on necessary pressure.

In addition, as shown in Fig. 3, multiple micro pumps can be arranged in parallel. Fig. 3 shows an example in which two micro pumps 131 and 132 are connected in parallel. By connecting the multiple micro pumps in parallel, flow amount of expelled gas can be increased.

Furthermore, as shown in Fig. 3, a structure is also possible in which a group in which multiple micro pumps are connected in series is prepared multiply, and the multiple groups are connected in parallel. Fig. 3 shows an example in which a group in which micro pumps 141 and 142 are connected in series and a group in which micro pumps 151 and 152 are connected in series are connected in parallel. By this structure, pressure and flow amount of expelled gas (which is sent) can be increased.

### Explanation of Reference Numerals

100: Ophthalmological device for measuring ocular pressure, 101: high pressure bomb, 102: pipe, 103: pressure regulator, 104: pressure storing tank (accumulator), 105: electromagnetic valve, 106: air chamber, 107: nozzle, 108: measuring optical system, 109: light transmitting part, 110: light transmitting part, 111: electromagnetic valve, 112: micro pump, 113: pressure sensor, 114: electromagnetic valve, 200: eye to be tested, 121: micro pump, 122: micro pump, 131: micro pump, 132: micro pump, 141: micro pump, 142: micro pump, 151: micro pump, 152: micro pump.

## Claims

1. An ophthalmological device comprising:
a high pressure gas supplying means for generating high pressure gas continuously,
a pressure storing tank for storing the high pressure gas supplied from the high pressure gas supplying means,
a pressure stabilizing means for maintaining an inner pressure of the pressure storing tank at a certain value,
a nozzle connected to the pressure storing tank and blowing the high pressure gas onto the eye of a subject, and
a primary ON-OFF valve arranged between the pressure storing tank and the nozzle.

2. The ophthalmological device according to claim 1, wherein the high pressure gas supplying means is a high pressure bomb storing high pressure gas.

3. The ophthalmological device according to claim 1, wherein the high pressure gas supplying means is a pump.

4. The ophthalmological device according to claim 3, wherein the pump generates high pressure air flow including pulsing flow using a diaphragm.

5. The ophthalmological device according to any one of claims 1 to 4, wherein high pressure gas is started to be blown onto the eye of a subject by making the primary ON-OFF valve opened, and then high pressure gas is stopped being blown onto the eye of a subject by making the primary ON-OFF valve closed.

6. The ophthalmological device according to any one of claims 1 to 5, wherein a secondary ON-OFF valve is arranged between the high pressure gas supplying means and the pressure storing tank; and high pressure gas is blown from the nozzle onto the eye of a subject by making an inside of the pressure storing tank be at a certain high pressure state under conditions in which the primary ON-OFF valve is closed and the secondary ON-OFF valve is opened, then, by making the secondary ON-OFF valve closed, and after that, by making the primary ON-OFF valve opened.

7. The ophthalmological device according to any one of claims 1 to 6, wherein a pressure sensor which detects inner pressure of the pressure storing tank and a third ON-OFF valve which opens and closes in order to maintain the inner pressure at a certain value, are arranged in the pressure storing tank.

8. The ophthalmological device according to any one of claims 3 to 7, wherein the pump comprises of multiple micro pumps, and the micro pumps are mutually connected in series and/or in parallel.
